# EUROPÄISCHE PATENTSCHRIFT

(11) **EP 4 142 817 B1**
(45) Veröffentlichungstag und Bekanntmachung des Hinweises auf die Patenterteilung: **23.10.2024**
(21) Anmeldenummer: 21722810.5
(22) Anmeldetag: 28.04.2021
(51) Int. Cl.: A61L 31/02, A61L 31/12

(54) **GRADIERTE DÜNNSCHICHTSYSTEME AUS METALL-KERAMIK-VERBUNDWERKSTOFFEN FÜR DIE BESCHICHTUNG KARDIOVASKULÄRER IMPLANTATE**
GRADED THIN LAYER SYSTEMS CONSISTING OF METAL-CERAMIC COMPOSITE MATERIALS FOR COATING CARDIOVASCULAR IMPLANTS
SYSTÈMES EN COUCHES MINCES À GRADIENT CONSTITUÉS DE MATÉRIAUX COMPOSITES MÉTAL-CÉRAMIQUE POUR LE REVÊTEMENT D'IMPLANTS CARDIOVASCULAIRES

(30) Priorität: 29.04.2020 DE 102020111669
(43) Veröffentlichungstag der Anmeldung: 08.03.2023
(73) Patentinhaber: Rheinisch-Westfälische Technische Hochschule (RWTH) Aachen, 52062 Aachen (DE)
(72) Erfinder: GRIGOREV, Nikita, 52072 Aachen (DE); SCHICKLE, Karolina, 52078 Aachen (DE); NEUSS-STEIN, Sabine, 52249 Eschweiler (DE); VOGT, Felix, 52074 Aachen (DE)
(74) Vertreter: Michalski Hüttermann & Partner Patentanwälte mbB
(86) Internationale Anmeldenummer: PCT/EP2021/061058
(87) Internationale Veröffentlichungsnummer: WO 2021/219687

(56) Entgegenhaltungen:
- EP-A1- 2 204 198
- EP-B1- 2 204 198
- DE-A1- 102010 027 532
- US-A1- 2007 264 303

## Beschreibung

Die Erfindung betrifft ein Verfahren zur Herstellung einer gradierten Metall-Keramik-Verbundwerkstoffbeschichtung auf einem metallischen kardiovaskulären Implantat und entsprechend hergestellte kardiovaskuläre Implantate, insbesondere Stents.

Stents sind im Allgemeinen röhrenförmige Vorrichtungen aus Metall oder Metalllegierungen wie Chrom-Nickel Legierung oder Kobaltbasislegierung (316L, L605), die im Rahmen der Behandlung von atherosklerotischen Stenosen in Blutgefäßen dazu dienen, einen Abschnitt des Gefäßes durchgängig zu halten. Stents sind üblicherweise hohle Gebilde mit gitterartigen, offenen Wandflächen, die im Gefäß expandiert werden können. Durch den Vorgang der Implantation des Stents sind Verletzungen der Gefäßwände unausweichlich. Diese können über die Aktivierung des Gerinnungssystems zu gefährlichen Stentthrombosen mit einem akuten Verschluss des Gefäßes führen, die mit einer Mortalität von etwa 45% assoziiert sind. Die aktuell vorwiegend verwendeten Drug Eluting Stents (DES) sind mit spezifischen Medikamenten wie Biolimus, Zotarolimus oder Everolimus beschichtet, die die zur Restenose führende neointimale Hyperplasie, d.h. die übermäßige Zellbildung innerhalb des Stents, hemmen können. Die Medikamente können direkt an der Oberfläche des Stents immobilisiert, in die porösen Strukturen an der Oberfläche infiltriert oder in eine resorbierbare Kunststoffbeschichtung integriert werden. Eine Restenose tritt dennoch abhängig vom Läsionstyp nach sechs Monaten mit einer Rate von bis zu 20% auf. Zur weiteren Reduktion der Raten von Stentthrombosen und Restenosen sind daher weitere Verbesserungen der Hämokompatibilität der verwendeten Materialien notwendig.

Verschiedene Beschichtungen von Stents sind im Stand der Technik bekannt. Beispielsweise beschreibt die US 5,496,359 A die Beschichtung von medizinischen Vorrichtungen wie Ballonstents mit Zirkonoxid oder Zirkonnitrid. Die US 5,649,951 A beschreibt ebenfalls mit Zirkonoxid oder Zirkonnitrid beschichtete Stents. Die DE 59609528 D1 beschreibt ein Verfahren zur Herstellung einer gradierten Beschichtung auf einem Metallimplantat durch ein elektrochemisches Verfahren. Hierbei ist das Implantat aus Titan oder Titanlegierungen ausgebildet und zur Verwendung als Dental-, Kiefer- oder Gelenkimplantat vorgesehen. Die DE 69627143 T2 beschreibt einen Metallstent mit einer darauf aufgebrachten blut- und biokompatiblen Schutzschicht, auf die weiter eine Metallbeschichtung aufgebracht wird, die die Röntgensichtbarkeit des Stents erhöhen soll. Eine direkte Beschichtung von metallischen Implantaten mit Keramik führt jedoch zu einer mangelhaften Haftung und ist für medizinische Anwendungen nicht geeignet. Zur Verbesserung der Haftung zwischen den Schichten werden in der DE 69627143 T2 Silanisierung, Säurebehandlung oder Sandstrahlen vorgeschlagen. Die DE 10 2020 020329 A1 beschreibt die galvanische Beschichtung eines Stents mit Magnesium.

Die DE 195 38 046 A1 beschreibt ein Schichtsystem aus einer Metallschicht, einer Keramikschicht und einer dazwischen liegenden gradierten Mischschicht aus Metall und Keramik. Als Anwendungsbeispiel sind lediglich C-Al-gradierte Schichten auf einem Siliziumwafer beschrieben, und die Schichtabscheidung erfolgt über Verfahren der Plasmabeschichtung. Derartige Verfahren sind jedoch für eine Herstellung von medizinischen Produkten ungeeignet und mit zu hohen Kosten verbunden.

US 2007/264303 A1 bezieht sich allgemein auf eine implantierbare medizinische Vorrichtung zur Abgabe eines therapeutischen Mittels an das Körpergewebe eines Patienten und auf ein Verfahren zur Herstellung einer solchen medizinischen Vorrichtung. Insbesondere wird eine implantierbare medizinische Vorrichtung, wie etwa ein intravaskulärer Stent, mit einer Beschichtung, die ein anorganisches oder keramisches Oxid, wie etwa Titanoxid, und ein therapeutisches Mittel umfasst, beschrieben.

Der vorliegenden Erfindung lag die Aufgabe zu Grunde, ein Verfahren für die Aufbringung einer gradierten Dünnschicht aus einem Metall-Keramik-Verbundwerkstoff auf ein metallisches Implantat zur Verfügung zu stellen, dass mindestens einen der vorgenannten Nachteile des Standes der Technik überwindet.

Diese Aufgabe wird gelöst durch ein Verfahren zur Herstellung einer gradierten Metall-Keramik-Verbundwerkstoffbeschichtung auf einem metallischen kardiovaskulären Implantat, wobei man die Verbundwerkstoffbeschichtung mittels eines hydrometallurgischen Verfahrens aus einer wässrigen Dispersion umfassend gelöstes Metallsalz oder eine gelöste Metallverbindung und dispergierte Keramikpartikel aufbringt. Die Aufgabe wird weiter gelöst durch ein metallisches kardiovaskuläres Implantat nach Anspruch 7.

Das Verfahren ermöglicht eine gleichmäßige, dünne und homogene Beschichtung der komplexen Oberfläche eines Implantats wie eines Stents mit einem Metall-Keramik-Verbundwerkstoff. Die Co-Abscheidung einer Metallmatrix mit keramischen Partikeln ermöglicht eine gradierte Metall-Keramik-Verbundwerkstoffbeschichtung in Form einer Dünnschicht auf kardiovaskulären Implantaten. Unter dem Begriff "kardiovaskuläre Implantate" werden Implantate mit Blutkontakt verstanden, beispielsweise Stents oder Herzklappen. Hierbei besorgt die Gradierung der Schicht eine bessere Anpassung und Anhaftung an das metallische Substrat. Eine galvanische Co-Abscheidung einer gradierten Metall-Keramik-Verbundwerkstoffbeschichtung direkt auf das Implantat erlaubt, die positiven Eigenschaften der Keramik hervorragend auszunutzen. Die gradierte Metall-Keramik-Verbundwerkstoffbeschichtung stellt damit eine verbesserte Hämokompatibilität und eine erhöhte Lebensdauer der beschichteten Implantate zur Verfügung, was zur Reduktion der Mortalität führen kann.

Der Begriff "hydrometallurgische Verfahren" umfasst als zusammenfassende Bezeichnung metallurgischen Verfahren zur Abscheidung von Metallen, insbesondere elektrochemische Verfahren (Elektrophorese) und Galvanisierung.

Hierbei bildet das metallische kardiovaskuläre Implantat die Substratelektrode und die wässrige Dispersion bildet den Elektrolyten, in den eine Gegenelektrode eingebracht wird. Durch einen gepulsten Strom und eine Variation des Tastgrades (Duty Cycle) können variable Anteile an Metall und Keramik und Gradierungen in der abgeschiedenen Beschichtung erzielt werden. Unter dem Begriff einer "Gradierung" einer Mischschicht wird verstanden, dass der Materialanteil einer Komponente in einer Richtung, beispielsweise der Wachstumsrichtung der Schicht, kontinuierlich abnimmt, während der Materialanteil der anderen Komponente gegenläufig zunimmt. Eine kontinuierliche Abnahme bedeutet, dass sprunghafte oder stufenförmige Änderungen der Konzentration vermieden sind. Vorliegend wird zunächst das Metall abgeschieden, so dass der Teil der Dünnschicht, der in direktem Kontakt mit dem Implantat steht, nahezu vollständig bzw. zu nahezu 100 % aus dem Metall ausgebildet wird. Dies ermöglicht das Anhaften der des Metall-Keramik-Verbunds an dem metallischen Implantat. Während der Abscheidung nimmt der Anteil von abgeschiedenem Metall in Wachstumsrichtung der Schicht, also von innen nach außen, ab, während der Anteil an abgeschiedenen Keramikpartikeln gegenläufig zunimmt. Die äußere Schicht des abgeschiedenen Metall-Keramik-Verbunds, die bei Verwendung in Kontakt mit Blut kommt, wird nahezu vollständig bzw. zu nahezu 100 % aus der Keramik ausgebildet. Hierdurch ist diese hämokompatibel.

In bevorzugten Ausführungsformen ist das Metall des Metallsalzes ausgewählt aus der Gruppe umfassend Nickel, Cobalt, Chrom, Molybdän, Eisen und deren Kombinationen. Eine bevorzugte Kombination der Metalle sind Legierungen wie Cobaltbasislegierungen mit Anteilen von Nickel, Chrom und Wolfram, beispielsweise L605, oder Cobalt-Chrom-Legierungen, insbesondere Ni-Co-Cr-Mo-Legierungen wie F562, und vergleichbare Legierungen. Hierbei kann das verwendete Metall dem Metall oder der Metalllegierungen entsprechend, aus dem das metallische kardiovaskuläre Implantat ausgebildet ist. Der Begriff "metallische" kardiovaskuläre Implantat schließt Metalllegierungen wie Stähle ein.

Verwendbar sind in wässriger Lösung lösliche organische und anorganische Salze und Verbindungen dieser Metalle. Bevorzugte Metallsalze sind in wässriger Lösung leicht lösliche Salze der Metalle wie Chloride oder Sulfate, die in Hydratform vorliegen können. In Ausführungsformen ist das Metallsalz ausgewählt aus der Gruppe umfassend Nickel(II)sulfat, Nickel(II)chlorid, Cobalt(II)sulfat, Cobalt(II)chlorid, Chrom(III)chlorid, Chrom(III)sulfat, Natriummolybdat, Eisen(II)-chlorid Hydrat, Eisen(II)-sulfat-hydrate, insbesondere Eisen(II)-sulfat-monohydrat, und/oder deren Kombinationen. Bevorzugt ist das Metallsalz ausgewählt aus der Gruppe umfassend Nickel(II)sulfat, Cobalt(II)sulfat und/oder Chrom(III)chlorid. Weiter können Metallverbindung wie Chromsäure (H₂CrO₄) eingesetzt werden oder sich in verdünnter wässriger Lösung ausbilden.

Die Konzentration der Metallsalze in der wässrigen Dispersion bzw. dem Elektrolyten kann variieren. In Ausführungsformen liegt die Konzentration des Metallsalzes oder der Metallsalze oder die Konzentration der Metallverbindung in der wässrigen Dispersion im Bereich von ≥ 10 g/L bis ≤ 500 g/L, im Bereich von ≥ 10 g/L bis ≤ 400 g/L, bevorzugt im Bereich von ≥ 30 g/L bis ≤ 300 g/L, weiter bevorzugt im Bereich von ≥ 60 g/L bis ≤ 300 g/L. zur Herstellung einer Matrix aus Nickel und Cobalt werden Nickel- und Cobaltsalze vorzugsweise in gleicher Konzentration im Elektrolyten eingesetzt, beispielweise in einer Konzentration von jeweils 60 g/L, so dass die Gesamtkonzentration der Salze vorzugsweise im Bereich von ≥ 100 g/L bis ≤ 200 g/L liegt. Die Konzentration der Chromsalze liegt vorzugsweise im Bereich von ≥ 100 g/L bis ≤ 300 g/L. Die Konzentration an Chromsalz kann drei bis vier Mal höher liegen als die Konzentration an Nickel- und/oder Cobaltsalzen.

Der pH-Wert der Dispersion bzw. des Elektrolyten liegt vorzugsweise im leicht sauren bis neutralen Bereich. In Ausführungsformen weist die wässrige Dispersion bzw. der Elektrolyt einen pH-Wert im Bereich von ≥ 2 bis ≤ 7, bevorzugt im Bereich von ≥ 2 bis ≤ 5, auf. In bevorzugten Ausführungsformen weist die wässrige Dispersion bzw. der Elektrolyt einen pH-Wert im Bereich von ≥ 1 bis ≤ 6, bevorzugt im Bereich von ≥ 1 bis ≤ 3, auf. Es konnte festgestellt werden, dass ein pH-Wert in diesen Bereichen das Abscheidungsverhalten positiv beeinflusst. Der pH-Wert kann beispielsweise mit Borsäure, Zitronensäure und/oder Ammoniak eingestellt werden.

In Ausführungsformen ist die Keramik ausgewählt aus der Gruppe umfassend Aluminiumoxid, Zirkonoxid, Siliziumcarbid und/oder Siliziumnitrid. Diese keramischen Werkstoffe zeichnen sich durch ihre hohen bioinerten Eigenschaften aus. Insbesondere Oxidkeramiken wie Aluminiumoxid (Al₂O₃) zeigen eine hohe Hämokompatibilität und Blutverträglichkeit. Auch Nichtoxidkeramiken wie Siliziumcarbid (SiC) in seinen verschiedenen polymorphen Modifikationen zeigen eine hervorragende Blutverträglichkeit. In Ausführungsformen liegt die Konzentration der Keramikpartikel in der wässrigen Dispersion im Bereich von ≥ 10 g/L bis ≤ 150 g/L, bevorzugt im Bereich von ≥ 30 g/L bis ≤ 100 g/L. Bei höheren Konzentrationen kann die Viskosität der Dispersion ansteigen und eine Abscheidung verlangsamen. In Ausführungsformen liegt die Konzentration an Al₂O₃-Partikel in der wässrigen Dispersion im Bereich von ≥ 10 g/L bis ≤ 150 g/L, bevorzugt im Bereich von ≥ 30 g/L bis ≤ 100 g/L. Es konnte festgestellt werden, dass Verbundschichten abgeschieden aus Dispersionen mit einer Konzentration von 30 g/L an Al₂O₃-Partikel eine geringere Zytotoxizität zeigte.

In Ausführungsformen weisen die Keramikpartikel eine mittle Partikelgröße im Bereich von ≥ 30 nm bis ≤ 100 nm, vorzugsweise im Bereich von ≥ 40 nm bis ≤ 100 nm, bevorzugt im Bereich von ≥ 50 nm bis ≤ 70 nm, auf. Partikel in diesen Größenbereichen können stabile kolloidale Systeme ausbilden. Größere Partikeln sedimentieren hingegen schneller, was für kolloidale Systeme und eine Co-abscheidung unterwünscht ist.

In bevorzugten Ausführungen zur Abscheidung einer Metall-Keramik-Verbundwerkstoffbeschichtung werden Cobaltsalz, Nickelsalz und Aluminiumoxidpartikel zur einer CoNi-Al₂O₃-Verbundwerkstoffbeschichtung abgeschieden. In weiter bevorzugten Ausführungen zur Abscheidung einer Metall-Keramik-Verbundwerkstoffbeschichtung werden Cobaltsalz, Nickelsalz, Chromsalz und Aluminiumoxidpartikel zur einer CoNiCr-Al₂O₃-Verbundwerkstoffbeschichtung oder Cobaltsalz, Nickelsalz, Chromsalz, Molybdänsalz und Aluminiumoxidpartikel zur einer CoNiCrMo-Al₂O₃-Verbundwerkstoffbeschichtung abgeschieden.

In einer Elektrolyseanordnung, in der die wässrige Dispersion den Elektrolyten bildet, dient das metallische kardiovaskuläre Implantat als Substratelektrode und kann mit einer geeigneten Gegenelektrode und Bezugselektrode kombiniert werden. Die Gradierung der Anteile an Metall und Keramik in der abgeschiedenen Verbundwerkstoffbeschichtung kann durch einen gepulsten Strom und eine Variation des Tastgrades erzielt werden. Die Variation des Tastgrades ist eine grundsätzlich bekannte digitaltechnische Methode zur Steuerung elektrischer Spannung und Leistung. Der Tastgrad (Duty Cycle) gibt hierbei für eine periodische Folge von Impulsen das Verhältnis der Impulsdauer (Torr) zur gesamt Periodendauer an und liegt in einem Werteberich zwischen 0 und 100%. Die Periodendauer setzt sich dabei aus den Einschaltzeiten (Torr) und den Pausenzeiten (T_{OFF}) zusammen und könnte periodisch über einen definierten Zeitraum, beispielsweise von 5 bis 10 Minuten erfolgen. Es werden Frequenzen beispielsweise zwischen 10 und 1000 Hz verwendet und eine bevorzugte Stromstärke liegt zwischen 0,1 und 0,3 A. Während der Impulsdauer (Torr) werden bevorzugt Metalle an der Gegenelektrode abgeschieden. Hingegen werden während der Ausschaltzeiten (T_{OFF}) vermehrt dispergierte Keramikpartikel in der Beschichtung eingebaut. Da über die Beschichtung vom Substrat zur Oberfläche hin der Anteil der eingebauten Partikel zunehmen soll, wird zu Beginn ein Tastverhältnis von 90% verwendet, danach wird dieses auf 50% reduziert und beträgt zum Schluss in der letzten Abscheidungsphase 10%. Die Gesamtdauer der Elektrolyse kann zwischen 15 und 60 Minuten variieren, vorzugsweise zwischen 15 und 30 Minuten. Die Temperatur kann zwischen 25 °C bis 60 °C, vorzugsweise zwischen 30-40 °C variieren.

Durch das beschriebene Verfahren lässt sich eine gradierte Metall-Keramik-Verbundwerkstoffbeschichtung aus der wässrigen Dispersion auf einem metallischen kardiovaskulären Implantat abscheiden. Die gradierte Metall-Keramik-Verbundwerkstoffbeschichtung bzw. Dünnschicht ist direkt auf dem Metall abgeschieden und umschließt vorzugsweise sämtliche Teile des Stents, die als kardiovaskuläre Prothese in den Körper eingebracht werden und dort verbleiben. Unter einer Dünnschicht wird eine Schicht mit einer Schichtdicke im Mikro- bzw. Nanometerbereich verstanden. Die gradierte Metall-Keramik-Verbundwerkstoffbeschichtung bewirkt, dass sich Blutpartikel nicht wie an einem metallischen Stent anhaften können. Hierdurch wird die Hämokompatibilität des Implantats verbessert und eine erhöhte Lebensdauer der beschichteten Implantate erzielt.

Ein weiterer Gegenstand der Erfindung betrifft ein metallisches kardiovaskuläres Implantat, wobei das Implantat eine gradierte Metall-Keramik-Verbundwerkstoffbeschichtung eines Metalls ausgewählt aus der Gruppe umfassend Nickel, Cobalt, Chrom, Molybdän, Eisen und deren Kombinationen und Partikeln einer Keramik ausgewählt aus der Gruppe umfassend Aluminiumoxid, Zirkonoxid, Siliziumcarbid und/oder Siliziumnitrid aufweist, hergestellt nach dem erfindungsgemäßen Verfahren. Für die weitere Beschreibung wird auf die voranstehenden Ausführungen zum Verfahren verwiesen.

Die gradierte Metall-Keramik-Verbundwerkstoffbeschichtung ist insbesondere als Dünnschicht ausgebildet. In der gradierten Metall-Keramik-Verbundwerkstoffbeschichtung nimmt der Materialanteil des Metalls ausgehend von der Oberfläche des metallischen Implantats nach außen hin kontinuierlich ab, während der Anteil an Keramikpartikeln gegenläufig zunimmt. Der Teil der Dünnschicht, der in direktem Kontakt mit dem Implantat steht, ist nahezu vollständig bzw. zu nahezu 100 % aus dem Metall ausgebildet. Dies ermöglicht das Anhaften der des Metall-Keramik-Verbunds an dem metallischen Implantat. Die äußere Schicht des Metall-Keramik-Verbunds, die bei Verwendung in Kontakt mit Blut kommt, wird nahezu vollständig bzw. zu nahezu 100 % aus der Keramik ausgebildet. Hierdurch ist die Oberfläche des beschichteten kardiovaskulären Implantats hämokompatibel.

Das metallische kardiovaskuläre Implantat ist vorzugsweise ein Stent oder eine Herzklappe. In bevorzugten Ausführungsformen ist das metallische kardiovaskuläre Implantat ein Stent.

In anderen Worten kann eine Verbundbeschichtung aus einer Metallmatrix mit integrierten, keramischen Nanopartikeln zur Verfügung gestellt werden, welche durch elektrolytische bzw. galvanische Koabscheidung aus einem Kolloid aus einer wässerigen Lösung von Metallsalzen und dispergierten, keramischen Nanopartikeln, realisiert wird.

Vorzugsweise ist die Keramik ausgewählt aus der Gruppe umfassend Aluminiumoxid, Zirkonoxid, Siliziumcarbid und/oder Siliziumnitrid. Diese keramischen Werkstoffe zeichnen sich durch ihre hohen bioinerten Eigenschaften aus. Insbesondere Oxidkeramiken wie Aluminiumoxid (Al₂O₃) zeigen eine hohe Hämokompatibilität und Blutverträglichkeit. Auch Nichtoxidkeramiken wie Siliziumcarbid (SiC) in seinen verschiedenen polymorphen Modifikationen zeigt eine hervorragende Blutverträglichkeit.

Bevorzugt ist eine Metallmatrix aus einer Kombination aus Nickel, Cobalt, Chrom, Molybdän und/oder Eisen mit eingebauten Keramikpartikeln, insbesondere aus Aluminiumoxid. Bevorzugt ist die Kombination der Metalle eine Legierung aus Nickel, Cobalt, Chrom, Molybdän und/oder Eisen. Eine bevorzugte Kombination der Metalle sind Legierungen wie Nickel-Cobalt-Chrom-Legierungen (NiCoCr), Cobalt-Chrom-Legierungen, Nickel-Chrom-Legierungen oder Nickel-Chrom-Molybdän-Cobalt-Legierungen. Hierbei kann das verwendete Metall dem Metall oder der Metalllegierungen entsprechend, aus dem das metallische kardiovaskuläre Implantat ausgebildet ist. Eine weitere bevorzugte Kombination der Metalle ist eine Nickel-Chrom-Eisen-Legierung (NiCrFe).

In bevorzugten Ausführungsformen ist die gradierte Metall-Keramik-Verbundwerkstoffbeschichtung ausgebildet aus Aluminiumoxid-Partikeln in einer Matrix aus Cobalt und Nickel oder Chrom und Cobalt, vorzugsweise in einer Matrix aus Cobalt, Nickel und Chrom. Eine CoNi-Al₂O₃-Metall-Keramik-Verbundwerkstoffbeschichtung zeigt besonders gute Flexibilität und Haftung. Weiterhin ist es möglich sehr dünne Schichten zu erzeugen.

Insgesamt kann ein Verfahren und eine gradierte Metall-Keramik-Verbundwerkstoffbeschichtung auf einem metallischen kardiovaskulären Implantat zur Verfügung gestellt werden, die eine verbesserte Hämokompatibilität und eine erhöhte Lebensdauer der beschichteten Implantate zur Verfügung stellen.

Wenn nicht anders ausgeführt, weisen die verwendeten technischen und wissenschaftlichen Ausdrücke die Bedeutung auf, wie sie gemeinhin von einem Durchschnittsfachmann in dem Gebiet, zu dem diese Erfindung gehört, verstanden wird.

Beispiele, die der Veranschaulichung der vorliegenden Erfindung dienen, sind nachstehend angegeben.

Hierbei zeigen die Figuren:
- Figur 1: zeigt einen Querschliff einer hydrometallurgisch mit Nickel beschichteten Gefäßprothese.
- Figur 2: zeigt eine Rasterelektronenmikroskop-Aufnahme der Oberfläche eines Edelstahl-Substrats mit einer Metall-Keramik-Verbundwerkstoffbeschichtung aus Nickel und Al₂O₃ besiedelt mit Zellen.
- Figur 3: zeigt eine Rasterelektronenmikroskop-Aufnahme der Oberfläche einer gradierten Metall-Keramik-Verbundwerkstoffbeschichtung aus Nickel mit Aluminiumoxid-Nanopartikeln.
- Figur 4: zeigt eine Rasterelektronenmikroskop-Aufnahme der Oberfläche einer gradierten Metall-Keramik-Verbundwerkstoffbeschichtung einer Nickel-Cobalt-Legierung mit Aluminiumoxid-Nanopartikeln.

### Beispiel 1

### Beschichtung einer Gefäßprothese mit Nickel

Eine kommerzielle Gefäßprothese (Medtronic Integrity CORONARY STENT System, 2,75 mm x 12 mm) wurde durch das Ballonträgersystem ausgedehnt und vom Trägersystem getrennt. Der Stent wurde für 3 min zur Aktivierung in 1M HCl-Lösung eingetaucht, danach mit bidestilliertem Wasser gespült und getrocknet.

Als Elektrolytlösung diente ein Nickelelektrolyt, der folgendermaßen hergestellt wurde: 240 g/L NiSO₄*6H₂O, 30 g/L NiCl₂*6H₂O und 30 g/L H₃BO₃ wurden in doppelt destilliertem Wasser unter ständigem Rühren mit einem Magnetrührer (300rpm) bei einer Temperatur von 50°C gelöst. Der pH-Wert des Elektrolyts wurde unter weiterer Zugabe von Borsäure auf pH 4 eingestellt. Die Gefäßprothese wurde als Kathode und reines Nickelblech als Anode mit 2 cm Abstand verwendet. Beide Elektroden wurden in den Elektrolyten eingetaucht. Die Stromstärke wurde auf 0,1 A eingestellt und die Beschichtungsdauer betrug 15 Minuten bei einer Temperatur von 50 °C. Die Probe wurde nach der Elektrolyse aus dem Elektrolyt rausgenommen und mit deionisiertem Wasser in Ultraschalbad für 15 min gereinigt und danach getrocknet.

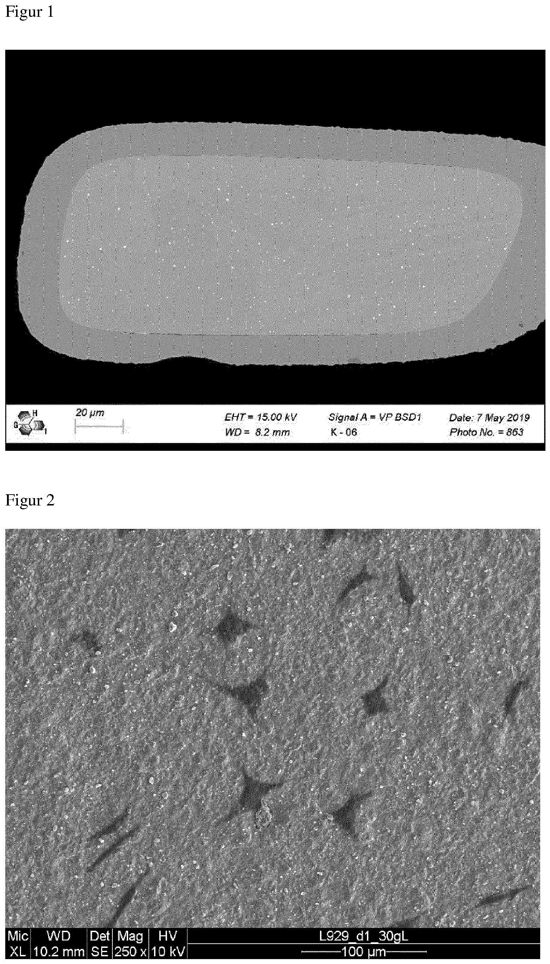

Die Figur 1 zeigt einen Querschliff der beschichteten Gefäßprothese. Wie man der Figur 1 entnimmt, wurde eine das kardiovaskuläre Implantat vollständig umschließende Dünnschicht aus Nickel abgeschieden.

### Beispiel 2

Abscheidung und zytotoxische Untersuchung einer gradierten Metall-Keramik-Verbundwerkstoffbeschichtung aus Nickel und Al₂O₃ auf einem Edelstahl-Substrat

### 2.1 Abscheidung einer gradierten Metall-Keramik-Verbundwerkstoffbeschichtung aus Nickel und Al₂O₃ auf einem Edelstahl-Substrat

Ein Edelstahl-Substrat aus 304L der Maße 10 x 10 x 2 mm wurde für 3 Minuten zur Aktivierung in 1M HCl Lösung eingetaucht und danach mit bidestilliertem Wasser gespült und getrocknet. Elektrolytlösungen enthaltend 240 g/L NiSO₄*6H₂O, und 30 g/L NiCl₂*6H₂O wurden wie in Beispiel 1 beschrieben hergestellt, wobei für zwei Proben eine weitere Zugabe von jeweils 15 g/L oder 30 g/L dispergierten Aluminiumoxid-Nanopartikeln (Aluminiumoxid, Nanopulver, 40-50nm, Alfa Aesar) erfolgte. Das Substrat, in einer Probenhalterung, wurde als Kathode und reines Nickelblech als Anode mit 2 cm Abstand verwendet. Alle Substrate wurden mit je drei Schichten bei den Tastgraden (Duty Cycle) 90% (erste Schicht), 50% (zweite Schicht) und 10% (dritte Schicht) für je 6 Minuten beschichtet. Die Frequenzzahl wurde auf 100 Hz eingestellt. Die Reinigung erfolgte in gleicher Weise wie in Beispiel 1 beschrieben.

### 2.2 Zytotoxische Untersuchung

Für die zytotoxische Untersuchung wurden die gemäß Beispiel 2.1 hergestellten Substrate nach der Elektrolyse für 3 Minuten mit 70% Ethanol gewaschen und mit Phosphat-gepufferter Salzlösung (Phosphate buffered saline, PBS) drei Mal gespült. Die Substrate wurden dann in 24-Kavitäten-Platten platziert.

Zytotoxizitätstests wurden gemäß ISO-Norm 10993/5 mit Zellen der Mausfibroblasten-Zelllinie L929 und humanen Endothelzellen (Human Coronary Artery Endothelial Cells, HCAEC) durchgeführt. Im Einzelnen wurde das Medium von L929- und HCAEC-Zellen abgesaugt und die Zellen wurden mit PBS gewaschen. Es wurden jeweils 3 ml Trypsin-EDTA-Lösung T 0,05 und 7 ml Medium hinzugegeben. Ein Teil der Zellen wurde mit CASY^{®}ton versehen und die Zellen wurden gezählt. Die restlichen Zellen im Medium wurden für 3 Minuten bei 220 g (9,81 m/s²) für HCAEC-Zellen bzw. 7 Minuten bei 1200 rpm für L929-Zellen zentrifugiert. Das Medium wurde abgesaugt, die Zellen wurden in 1 ml Medium resuspendiert und Plasmocin wurde hinzugegeben. Pro Quadratzentimeter des beschichteten Substrats wurden 10000 Zellen eingesetzt. Die Proben wurden 24 Stunden im Inkubator gelagert. Anschließend wurden die Zellen mit einer Färbelösung aus 600 µl Ringerlösung, 5 ng/ml Fluoresceiniacetat (FDA) und 5ng/ml Propidiumiodid (PI7) eingefärbt und gezählt. Die folgenden Tabellen 1 und 2 zeigen die Ergebnisse der Zytotoxizitätstests.

**Tabelle 1: Ergebnisse der Zytotoxizitätstest mit L929-Zellen:**

| Beschichtung des Substrats | Zell-Mortalität |
|---|---|
| Nickel | 8,7 % |
| Nickel-Matrix mit dispergiertem Al₂O₃ (15 g/L) | 2,87 % |
| Nickel-Matrix mit dispergiertem Al₂O₃ (30 g/L) | 2,15 % |

**Tabelle 2: Ergebnisse der Zytotoxizitätstest mit HCAEC-Zellen:**

| Beschichtung des Substrats | Zell-Mortalität |
|---|---|
| Nickel | 10,29 % |
| Nickel-Matrix mit dispergiertem Al₂O₃ (15 g/L) | 1,65 % |
| Nickel-Matrix mit dispergiertem Al₂O₃ (30 g/L) | 2,41 % |

Wie man den Ergebnissen der Tabellen 1 und 2 entnimmt, zeigte ein steigender Keramikanteil einen deutlich positiven Einfluss auf das Zellenverhalten. Dies zeigt, dass die Zytokompatibilität mit steigendem Keramikanteil zunimmt.

Die Figur 2 zeigt eine mikroskopische Aufnahme (Rasterelektronenmikroskop, Environmental Scanning Electron Microscope (ESEM XL30 FEG, FEI, Eindhoven, Niederlande)) der Oberfläche des Edelstahl-Substrats beschichtet mit einer Nickel-Matrix mit dispergiertem Al₂O₃ (30 g/L) besiedelt mit HCAEC-Zellen nach 24 Stunden Inkubation. Lichtmikroskopische Aufnahmen bestätigten, dass nach 24 Stunden Inkubation die Zellen auf den Substraten beschichtet mit höherem Aluminiumoxidgehalt deutlich vitaler wirkten.

### Beispiel 3

### Abscheidung einer gradierten Metall-Keramik-Verbundwerkstoffbeschichtung aus Nickel und Al₂O₃ auf einem Edelstahl-Substrat

Ein Edelstahl-Substrat aus 304L der Maße 50x20x2 [mm] wurde in einem Ultraschallbad für 30 min gereinigt, für 3 Minuten zur Aktivierung in 1M HCl-Lösung eingetaucht und danach mit bidestilliertem Wasser gespült und getrocknet. Eine Elektrolytlösung enthaltend 240 g/L NiSO₄*6H₂O,und 30 g/L NiCl₂*6H₂O wurde wie in Beispiel 1 beschrieben hergestellt, wobei 100 g/L dispergierte Aluminiumoxid-Nanopartikel (Aluminiumoxid, Nanopulver, 40-50nm, Alfa Aesar) zugegeben wurde.

Das Substrat, in einer Probenhalterung, wurde als Kathode und reines Nickelblech als Anode mit 2 cm Abstand verwendet. Das Substrat wurden mit je drei Schichten bei den Tastgraden (Duty Cycle) 90% (erste Schicht), 50% (zweite Schicht) und 10% (dritte Schicht) bei 100Hz, und bei einer Spitzenstromstärke von 0,3 A für 6 Minuten pro Schicht, also 18 Minuten insgesamt, beschichtet. Die Reinigung des beschichteten Substrates erfolgt in gleicher Weise wie in Beispiel 1 beschrieben.

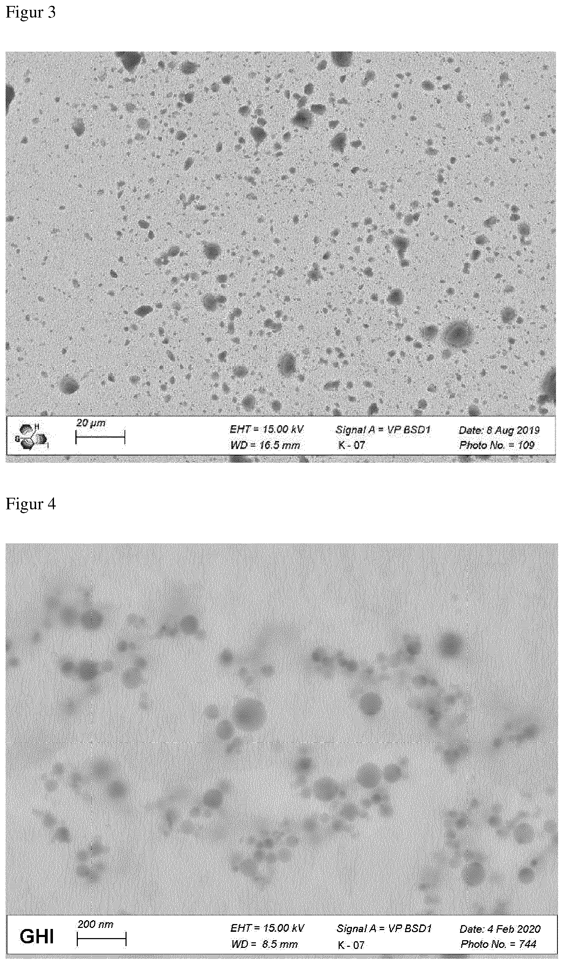

Die Figur 3 zeigt eine Rasterelektronenmikroskop-Aufnahme (Gemini SEM 500, ZEISS) der Oberfläche der Beschichtung mit einer Metallmatrix mit eingebauten Aluminiumoxid-Nanopartikeln. Anhand der Aufnahme ist zu erkennen, dass Aluminiumoxidpartikel an der Probenoberfläche in die Metallmatrix eingebaut sind. Diese sind in der Abbildung als dunkle Flecken zu erkennen.

### Beispiel 4

### Abscheidung einer gradierten Metall-Keramik-Verbundwerkstoffbeschichtung aus einer Nickel-Cobalt-Legierung und Al₂O₃ auf einem Edelstahl-Substrat

### 4.1 Vorversuch: Beschichtung eines Edelstahl-Substrats mit einer Nickel-Cobalt-Legierung

Ein Edelstahl-Substrat aus 304L der Maße 50x20x2 [mm] wurde in einem Ultraschallbad für 30 min gereinigt, für 3 Minuten zur Aktivierung in 1M HCl-Lösung eingetaucht und danach mit bidestilliertem Wasser gespült und getrocknet. Als Elektrolyt dient ein Nickel-Cobalt-Elektrolyt, das folgendermaßen hergestellt wurde: 130 g/L CoSO₄*7H₂O, 130 g/L NiSO₄*6H₂O und 30 g/L H₃BO₃ wurden in doppelt destilliertem Wasser unter ständigem Rühren mit einem Magnetrührer (150 rpm) bei einer Temperatur von 35°C gelöst. Der pH-Wert des Elektrolyts wurde auf pH 5 unter Zugabe von NH₃-Lösung eingestellt. Das Substrat, in einer Probenhalterung, wurde als Kathode und reines Nickelblech als Anode mit 2 cm Abstand verwendet. Die Stromstärke wurde auf 0,2 A eingestellt und die Beschichtungsdauer betrug 12 Minuten. Die Reinigung erfolgt in gleicher Weise wie in Beispiel 1 beschrieben.

Durch Röntgenspektroskopie (EDX-Analyse) der beschichteten Oberfläche (Gemini SEM 500, ZEISS) wurde ermittelt, dass die aufgebrachte NiCo-Legierung 86,3 Gew.% Nickel und 13,5 Gew.% Cobalt enthielt.

### 4.2 Abscheidung einer gradierten Metall-Keramik-Verbundwerkstoffbeschichtung aus einer Nickel-Cobalt-Legierung und Al₂O₃

Ein Edelstahl-Substrat aus 304L der Maße 50x20x2 [mm] wurde in einem Ultraschallbad für 30 min gereinigt, für 3 Minuten zur Aktivierung in 1M HCl-Lösung eingetaucht und danach mit bidestilliertem Wasser gespült und getrocknet. Als Elektrolyt dient ein Nickel-Kobalt-Elektrolyt, das folgendermaßen hergestellt wurde: 130 g/L CoSO₄*7H₂O, 130 g/L NiSO₄*6H₂O und 30 g/L H₃BO₃ und 10 g/L Al₂O₃ wurden in doppelt destilliertem Wasser unter ständigem Rühren mit einem Magnetrührer (150 rpm) bei einer Temperatur von 35°C gelöst. Der pH-Wert des Elektrolyts wurde auf pH 5 unter Zugabe von NH₃-Lösung eingestellt. Das Substrat, in einer Probenhalterung, wurde als Kathode und reines Nickelblech als Anode mit 2 cm Abstand verwendet. Die Stromstärke wurde auf 0,2 A eingestellt und die Beschichtungsdauer betrug 12 Minuten. Die Reinigung erfolgt in gleicher Weise wie in Beispiel 1 beschrieben.

Die Figur 4 zeigt eine Rasterelektronenmikroskop-Aufnahme (Gemini SEM 500, ZEISS) der Oberfläche (Querschliff) der Beschichtung mit einer Nickel-Cobaltmatrix mit eingebauten Aluminiumoxid-Nanopartikeln. Anhand der Aufnahme ist zu erkennen, dass Aluminiumoxidpartikel an der Probenoberfläche in die Metallmatrix eingebaut wurden. Die Aluminiumoxidpartikel sind in der Abbildung als dunkle Flecken zu erkennen.

### Beispiel 5

### Beschichtung eines Edelstahl-Substrats mit einer Nickel-Cobalt-Chrom-Legierung

Ein Substrat aus 304L der Maße 50x20x2 [mm] wurde in einem Ultraschallbad für 30 min gereinigt, für 3 Minuten in 1M HCl-Lösung eingetaucht und danach mit bidestilliertem Wasser gespült und getrocknet. Als Elektrolyt dient ein Nickel-Cobalt-Chrom-Elektrolyt folgender Zusammensetzung: 60 g/L CoSO₄*7H₂O, 60 g/L NiSO₄*6H₂O, 120 g/L CrCl₃*6H₂O, und 30 g/L H₃BO₃. Die Chemikalien wurden unter ständigem Rühren bei einer Temperatur von etwa 65°C gelöst. Der pH-Wert wurde mit einer NH₃-Lösung und Borsäure auf einen Wert von pH 2 eingestellt. Das Substrat, in einer Probenhalterung, wurde als Kathode und reines Nickelblech als Anode mit 2 cm Abstand verwendet. Die Stromstärke wurde auf 0,1 A eingestellt und die Beschichtungsdauer betrug 30 Minuten. Die Reinigung erfolgt in gleicher Weise wie in Beispiel 1 beschrieben.

Durch Röntgenspektroskopie (EDX-Analyse, Gemini SEM 500, ZEISS) der beschichteten Oberfläche wurde festgestellt, dass die Metallzusammensetzung der abgeschiedenen NiCoCr-Legierung betrug 14,5 Gew.% Nickel, 78,4 Gew.% Cobalt und 1,4 Gew.% Chrom.

## Patentansprüche

1. Verfahren zur Herstellung einer gradierten Metall-Keramik-Verbundwerkstoffbeschichtung auf einem metallischen kardiovaskulären Implantat, **dadurch gekennzeichnet, dass** man die Verbundwerkstoffbeschichtung mittels eines hydrometallurgischen Verfahrens aus einer wässrigen Dispersion umfassend gelöstes Metallsalz oder eine gelöste Metallverbindung und dispergierte Keramikpartikel aufbringt.

2. Verfahren nach Anspruch 1, **dadurch gekennzeichnet, dass** das Metall des Metallsalzes ausgewählt ist aus der Gruppe umfassend Nickel, Cobalt, Chrom, Molybdän, Eisen und deren Kombinationen.

3. Verfahren nach Anspruch 1 oder 2, **dadurch gekennzeichnet, dass** die Konzentration des oder der Metallsalze(s) oder der Metallverbindung in der wässrigen Dispersion im Bereich von ≥ 10 g/L bis ≤ 500 g/L, vorzugsweise im Bereich von ≥ 10 g/L bis ≤ 400 g/L, bevorzugt im Bereich von ≥ 30 g/L bis ≤ 300 g/L, liegt.

4. Verfahren nach einem der vorhergehenden Ansprüche, **dadurch gekennzeichnet, dass** die wässrige Dispersion einen pH-Wert im Bereich von ≥ 2 bis ≤ 7, bevorzugt im Bereich von≥ 2 bis ≤ 5, aufweist.

5. Verfahren nach einem der vorhergehenden Ansprüche, **dadurch gekennzeichnet, dass** die Keramik ausgewählt ist aus der Gruppe umfassend Aluminiumoxid, Zirkonoxid, Siliziumcarbid und/oder Siliziumnitrid.

6. Verfahren nach einem der vorhergehenden Ansprüche, **dadurch gekennzeichnet, dass** die Keramikpartikel eine mittle Partikelgröße im Bereich von ≥ 30 nm bis ≤ 100 nm, vorzugsweise im Bereich von ≥ 40 nm bis ≤ 100 nm, bevorzugt im Bereich von ≥ 50 nm bis ≤ 70 nm, aufweisen.

7. Metallisches kardiovaskuläres Implantat, wobei das Implantat eine gradierte Metall-Keramik-Verbundwerkstoffbeschichtung eines Metalls ausgewählt aus der Gruppe umfassend Nickel, Cobalt, Chrom, Molybdän, Eisen und deren Kombinationen und Partikeln einer Keramik ausgewählt aus der Gruppe umfassend Aluminiumoxid, Zirkonoxid, Siliziumcarbid und/oder Siliziumnitrid aufweist, wobei in der gradierten Metall-Keramik-Verbundwerkstoffbeschichtung der Materialanteil des Metalls ausgehend von der Oberfläche des metallischen Implantats nach außen hin kontinuierlich abnimmt, während der Anteil an Keramikpartikeln gegenläufig zunimmt, hergestellt nach einem Verfahren gemäß einem der Ansprüche 1 bis 6.

8. Implantat nach Anspruch 7, **dadurch gekennzeichnet, dass** das metallische kardiovaskuläre Implantat ein Stent ist.

9. Implantat nach Anspruch 7 oder 8, **dadurch gekennzeichnet, dass** die Kombination der Metalle eine Legierung ausgebildet aus Nickel, Cobalt, Chrom, Molybdän und/oder Eisen ist.

10. Implantat nach einem der Ansprüche 7 bis 9, **dadurch gekennzeichnet, dass** die Kombination der Metalle eine Legierung ist ausgewählt aus Nickel-Cobalt-Chrom-Legierungen, Cobalt-Chrom-Legierungen, Nickel-Chrom-Legierungen oder Nickel-Chrom-Molybdän-Cobalt-Legierungen.

11. Implantat nach einem der Ansprüche 7 bis 10, **dadurch gekennzeichnet, dass** die Kombination der Metalle eine Nickel-Chrom-Eisen-Legierung ist.

12. Implantat nach einem der Ansprüche 7 bis 11, **dadurch gekennzeichnet, dass** die gradierte Metall-Keramik-Verbundwerkstoffbeschichtung ausgebildet ist aus Aluminiumoxid-Partikeln in einer Matrix aus Cobalt und Nickel oder Chrom und Cobalt, vorzugsweise in einer Matrix aus Cobalt, Nickel und Chrom.

## Claims

1. Method for producing a graded metal-ceramic composite coating on a metallic cardiovascular implant, **characterized in that** the composite coating is applied via a hydrometallurgical method from an aqueous dispersion comprising dissolved metal salt or a dissolved metal compound and dispersed ceramic particles.

2. Method according to Claim 1, **characterized in that** the metal of the metal salt is selected from the group comprising nickel, cobalt, chromium, molybdenum, iron and combinations thereof.

3. Method according to Claim 1 or 2, **characterized in that** the concentration of the metal salt or salts or of the metal compound in the aqueous dispersion is in the range from ≥ 10 g/L to ≤ 500 g/L, preferably in the range from ≥ 10 g/L to ≤ 400 g/L, more preferably in the range from ≥ 30 g/L to ≤ 300 g/L.

4. Method according to any of the preceding claims, **characterized in that** the aqueous dispersion has a pH in the range from ≥ 2 to ≤ 7, more preferably in the range from ≥ 2 to ≤ 5.

5. Method according to any of the preceding claims, **characterized in that** the ceramic is selected from the group comprising aluminium oxide, zirconium oxide, silicon carbide and/or silicon nitride.

6. Method according to any of the preceding claims, **characterized in that** the ceramic particles have a mean particle size in the range from ≥ 30 nm to ≤ 100 nm, preferably in the range from ≥ 40 nm to ≤ 100 nm, more preferably in the range from ≥ 50 nm to ≤ 70 nm.

7. Metallic cardiovascular implant, the implant comprising a graded metal-ceramic composite coating of a metal selected from the group encompassing nickel, cobalt, chromium, molybdenum, iron and combinations thereof and particles of a ceramic selected from the group encompassing aluminium oxide, zirconium oxide, silicon carbide and/or silicon nitride, wherein the materials fraction of the metal decreases continuously from the surface of the metallic implant towards the outside in the graded metal-ceramic composite coating, while the fraction of ceramic particles increases in the opposite direction, produced by a method according to any of Claims 1 to 6.

8. Implant according to Claim 7, **characterized in that** the metallic cardiovascular implant is a stent.

9. Implant according to Claim 7 or 8, **characterized in that** the combination of the metals is an alloy formed of nickel, cobalt, chromium, molybdenum and/or iron.

10. Implant according to any of Claims 7 to 9, **characterized in that** the combination of the metals is an alloy selected from nickel-cobalt-chromium alloys, cobalt-chromium alloys, nickel-chromium alloys or nickel-chromium-molybdenum-cobalt alloys.

11. Implant according to any of Claims 7 to 10, **characterized in that** the combination of the metals is a nickel-chromium-iron alloy.

12. Implant according to any of Claims 7 to 11, **characterized in that** the graded metal-ceramic composite coating is formed of aluminium oxide particles in a matrix of cobalt and nickel or chromium and cobalt, preferably in a matrix of cobalt, nickel and chromium.

## Revendications

1. Procédé de fabrication d'un revêtement en matériau composite métal-céramique à gradient sur un implant cardiovasculaire métallique, **caractérisé en ce qu'**on applique le revêtement de matériau composite par un procédé hydrométallurgique à partir d'une dispersion aqueuse comprenant un sel métallique dissous ou un composé métallique dissous et des particules céramiques dispersées.

2. Procédé selon la revendication 1, **caractérisé en ce que** le métal du sel métallique est choisi dans le groupe comprenant les métaux nickel, cobalt, chrome, molybdène, fer et leurs combinaisons.

3. Procédé selon la revendication 1 ou 2, **caractérisé en ce que** la concentration du ou des sels métalliques ou du composé métallique dans la dispersion aqueuse est comprise dans la plage de ≥ 10 g/L à < 500 g/L, de préférence dans la plage de ≥ 10 g/L à ≤ 400 g/L, préférentiellement dans la plage de ≥ 30 g/L à ≤ 300 g/L.

4. Procédé selon l'une des revendications précédentes, **caractérisé en ce que** la dispersion aqueuse présente un pH dans la plage de ≥ 2 à ≤ 7, préférentiellement dans la plage de ≥ 2 à ≤ 5.

5. Procédé selon l'une des revendications précédentes, **caractérisé en ce que** la céramique est choisie dans le groupe comprenant l'oxyde d'aluminium, l'oxyde de zirconium, le carbure de silicium et/ou le nitrure de silicium.

6. Procédé selon l'une des revendications précédentes, **caractérisé en ce que** les particules céramiques présentent une granulométrie moyenne dans la plage de ≥ 30 nm à ≤ 100 nm, de préférence dans la plage de ≥ 40 nm à ≤ 100 nm, préférentiellement dans la plage de ≥ 50 nm à ≤ 70 nm.

7. Implant cardiovasculaire métallique, l'implant comportant un revêtement de matériau composite métal-céramique gradient d'un métal choisi dans le groupe comprenant les métaux nickel, cobalt, chrome, molybdène, fer et leurs combinaisons et des particules d'une céramique choisie dans le groupe comprenant l'oxyde d'aluminium, l'oxyde de zirconium, le carbure de silicium et/ou le nitrure de silicium, la proportion du matériau métal, dans le revêtement de matériau composite métal-céramique à gradient, en partant de la surface de l'implant métallique vers l'extérieur, diminuant en continu, tandis que la proportion des particules céramiques augmente en sens inverse, fabriqué par un procédé selon l'une des revendications 1 à 6.

8. Implant selon la revendication 7, **caractérisé en ce que** l'implant cardiovasculaire métallique est un stent.

9. Implant selon la revendication 7 ou 8, **caractérisé en ce que** la combinaison des métaux est un alliage formé à partir de nickel, de cobalt, de chrome, de molybdène et/ou de fer.

10. Implant selon l'une des revendications 7 à 9, **caractérisé en ce que** la combinaison des métaux est un alliage choisi parmi les alliages nickel-cobalt-chrome, les alliages cobalt-chrome, les alliages nickel-chrome ou les alliages nickel-chrome-molybdène-cobalt.

11. Implant selon l'une des revendications 7 à 10, **caractérisé en ce que** la combinaison des métaux est un alliage nickel-chrome-fer.

12. Implant selon l'une des revendications 7 à 11, **caractérisé en ce que** le revêtement de matériau composite métal-céramique à gradient est formé à partir de particules d'oxyde d'aluminium dans une matrice de cobalt et de nickel ou de chrome et de cobalt, de préférence dans une matrice de cobalt, de nickel et de chrome.
